# FASCICULE DE BREVET EUROPEEN

(11) **EP 3 389 679 B1**
(45) Date de publication et mention de la délivrance du brevet: **12.02.2020**
(21) Numéro de dépôt: 16826115.4
(22) Date de dépôt: 19.12.2016
(51) Int. Cl.: A61K 35/37, C12N 1/04, C12Q 1/02, A61K 47/10, A61K 35/74, A61P 1/00

(54) **PROCEDE DE LYOPHILISATION D'UN ECHANTILLON DE MICROBIOTE FECAL**
VERFAHREN ZUR LYOPHILISIERUNG EINER FÄKALEN MIKROBIOTAPROBE
METHOD FOR LYOPHILISATION OF A SAMPLE OF FECAL MICROBIOTA

(30) Priorité: 18.12.2015 FR 1562750
(43) Date de publication de la demande: 24.10.2018
(73) Titulaire: Maat Pharma, 69007 Lyon (FR); Institut National De La Recherche Agronomique, 75007 Paris (FR)
(72) Inventeur: AFFAGARD, Hervé, 69007 Lyon (FR); SCHWINTNER, Carole, 69007 Lyon (FR); JUSTE, Catherine, 75007 Paris (FR); DORE, Joël, 75007 Paris (FR); CHAPRON, Audrey, 75007 Paris (FR); FONSECA, Fernanda, 75007 Paris (FR); DAVID, Olivier, 75007 Paris (FR)
(74) Mandataire: Santarelli
(86) Numéro de dépôt international: PCT/FR2016/053550
(87) Numéro de publication internationale: WO 2017/103550

(56) Documents cités:
- WO-A1-2014/152484
- US-A1- 2014 147 417
- US-A1- 2014 328 803
- US-A1- 2015 044 173
- HAMILTON MATTHEW J ET AL: "Standardized Frozen Preparation for Transplantation of Fecal Microbiota for Recurrent Clostridium difficile Infection", AMERICAN JOURNAL OF GASTROENTEROLOGY, ELSEVIER SCIENCE INC, US, vol. 107, no. 5, 1 mai 2012 (2012-05-01), pages 761-767, XP009161359, ISSN: 0002-9270, DOI: 10.1038/AJG.2011.482
- Anonymous: "Lyophilisation - Wikipédia", , 10 septembre 2015 (2015-09-10), XP055302259, Extrait de l'Internet: URL:https://web.archive.org/web/2015091012 5419/https://fr.wikipedia.org/wiki/Lyophil isation [extrait le 2016-09-13]

## Description

La présente invention se rapporte à un procédé de lyophilisation d'un échantillon de microbiote fécal. L'invention se rapporte également à l'utilisation du lyophilisat obtenu dans la transplantation de microbiote fécal, de préférence pour traiter les dysbioses intestinales, notamment les infections à *Clostridium difficile.*

Le microbiote intestinal humain est l'ensemble des micro-organismes (bactéries, levures et champignons) qui se trouvent dans le système gastro-intestinal humain (estomac, intestin et côlon). La diversité microbienne est estimée à l'heure actuelle à environ 10³ espèces bactériennes composant le microbiote intestinal dominant d'un individu adulte, avec une abondance de 10¹⁴ bactéries, représentant un métagénome bactérien dominant de 200 000 à 800 000 gènes chez chaque individu, soit 10 à 50 fois le nombre de gènes du génome humain.

Stérile *in utero,* l'intestin se colonise dès les premiers jours de vie jusqu'à évoluer vers un microbiote individuel unique. Chaque personne possède des bactéries relativement proches en termes d'espèces, mais la composition exacte de son microbiote (espèces, proportions) est pour une large part (plus de ²/₃ des espèces) spécifique de l'hôte.

Ainsi, le microbiote intestinal humain est un écosystème très diversifié, complexe et spécifique de chaque individu.

Il est essentiel pour la santé d'un individu de maintenir un microbiote stable qui est à la fois capable de revenir à son état initial après un changement et résistant à l'invasion. Le maintien d'une grande diversité du microbiote favorise sa stabilité. Cependant, certaines pathologies ou traitements déséquilibrent le microbiote : les antibiotiques par exemple, ainsi que les maladies à composante inflammatoire, telle que les maladies inflammatoires chroniques de l'intestin (MICI), peuvent limiter la diversité du microbiote dans l'intestin.

Les traitements antibiotiques (ou antibiothérapie), notamment, résultent en une altération du microbiote et une perte de ses fonctions de barrière, ce qui peut favoriser la prolifération d'organismes pathogènes comme *Clostridium difficile.*

Les infections à *Clostridium difficile* sont responsables de diarrhées nosocomiales ; cette bactérie peut présenter des résistances à l'antibiothérapie classique (à spectre large, tels que vancomycine ou métronidazole). Afin de rétablir le microbiote intestinal, et lutter contre les infections de type *Clostridium difficile,* et ainsi rétablir l'homéostasie (*i.e.* la symbiose), la transplantation de microbiote fécal est envisagée et testée. Elle consiste en l'introduction des selles d'un sujet donneur sain dans le tube digestif d'un patient receveur, afin de rééquilibrer le microbiote intestinal altérée de l'hôte. Cette transplantation de microbiote fécal est en général allogénique (c'est-à-dire d'un individu donneur sain vers un patient). Les résultats obtenus sur les infections de type *Clostridium difficile* sont encourageants, et certains patients ont été traités avec succès (Tauxe et al, Lab Medicine, Winter 2015, volume 46, Number 1 ou van Nood E, Speelman P, Nieuwdorp M, Keller J. 2014 Fecal microbiota transplantation: facts and controversies. Curr Opin Gastroenterol 30(1):34-9).

Cependant, la méthode de transplantation actuelle est empirique et ne prend aucune précaution particulière pour préserver au mieux la viabilité des bactéries anaérobies, composants majoritaires du microbiote intestinal. En outre, l'efficacité de la transplantation de microbiote fécal est variable, et peut nécessiter plus d'une cure.

Il existe donc un besoin de disposer d'échantillons de microbiote fécal sécurisés, efficaces et faciles à obtenir, notamment à l'échelle industrielle. En outre, il existe un besoin pour des échantillons de microbiote fécal dans lesquels la viabilité des bactéries est conservée, et ayant une longue durée de conservation.

La présente invention permet de répondre à ces besoins.

La présente invention se rapporte donc à un procédé de préparation d'un lyophilisat de microbiote fécal d'un sujet donneur, comprenant les étapes suivantes :
A) le mélange d'un échantillon de microbiote fécal d'un sujet donneur avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A) à une température inférieure à -50°C, de préférence comprise entre -70°C et -100°C, puis sa lyophilisation.

Dans un autre mode de réalisation qui peut être combiné avec le mode de realisation précédent, le diluent est une solution aqueuse saline comprenant au moins le galactose-lactose ou le tréhalose en tant que cryoprotectant.

Dans un autre mode de réalisation qui peut être combiné avec l'un des modes de réalisation précédents, le diluant est une solution aqueuse saline comprenant au moins un mélange de maltodextrines comme agent de charge, de préférence dans une quantité comprise entre 4 et 20% par rapport au volume total de la solution.

Dans un autre mode de réalisation qui peut être combiné avec l'un des modes précédents, la quantité totale de cryoprotectant dans la solution aqueuse saline est comprise entre 3 et 30% en poids par rapport au volume total de la solution, de préférence entre 4 et 20% en poids par rapport au volume totale de la solution.

De préférence, l'échantillon de microbiote fécal utilisé à l'étape A) est préalablement purifié.

De préférence, la présente invention se rapporte à un procédé de préparation d'un lyophilisat de microbiote fécal d'un sujet donneur, comprenant les étapes suivantes :
A1) optionnellement, la préparation d'un gradient continu d'iodixanol ou de 5- (N-2, 3-dihydroxypropylacetamido)-2, 4, 6-tri-iodo-N, N'-bis (2, 3 dihydroxypropyl) isophthalamide formé par congélation-décongélation,
A2) le mélange d'au moins un échantillon de microbiote fécal d'un sujet donneur avec un tampon salin comprenant éventuellement de l'iodixanol ou du 5- (N-2, 3-dihydroxypropylacetamido)-2, 4, 6-tri-iodo-N, N'-bis (2, 3 dihydroxypropyl) isophthalamide, en anaérobiose,
A3) optionnellement, le dépôt du mélange obtenu en A2) sous le gradient obtenu en A1),
A4) la centrifugation séquentielle à faible accélération en anaérobiose, ou l'ultracentrifugation du mélange obtenu en A2) ou A3),
A5) la récupération de l'anneau bactérien ou du surnageant formé à l'issue de l'étape A4), en anaérobiose,
A6) le mélange de l'anneau bactérien ou du surnageant récupéré en A5) avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A6) à une température inférieure à -50°C, de préférence comprise entre -70°C et -100°C, puis sa lyophilisation.

Dans un tel procédé, l'étape A1), ainsi que *de facto* l'étape A3), sont facultatives.

Un tel procédé est en effet facile à mettre en œuvre, et son efficacité peut être estimée en comparant la population microbienne obtenue après le procédé, comparée au prélèvement initial.

La présente invention se rapporte également à l'utilisation d'un lyophilisat de microbiote fécal susceptible d'être obtenu par le procédé selon l'invention, comme outil de recherche en génomique fonctionnelle, en métaprotéomique ou en immunologie.

Par exemple, le lyophilisat susceptible d'être obtenu par le procédé selon l'invention peut être utilisé pour générer des culots bactériens qui, immobilisés dans des matrices d'agarose (ou autres gels), permettent l'extraction de fragments d'ADN de très grande taille, utilisés pour du clonage et des études fonctionnelles. Il peut être utilisé également pour préparer des extraits de protéines cytosoliques ou de protéines d'enveloppes de communautés bactériennes en vue d'analyses métaprotéomiques. Enfin, il peut être utilisé pour étudier la reconnaissance des bactéries intactes par le système immunitaire de l'hôte.

La présente invention se rapporte également à l'utilisation d'un lyophilisat de microbiote fécal d'un sujet donneur susceptible d'être obtenu par le procédé selon l'invention, dans la transplantation de microbiote fécal autologue ou allogénique.

La présente invention se rapporte également à l'utilisation d'un lyophilisat de microbiote fécal d'un sujet donneur susceptible d'être obtenu par le procédé selon l'invention, pour traiter les dysbioses intestinales, dues à des infections et notamment les infections à *Clostridium difficile,* les dysbioses induites par des traitements médicamenteux, par des traitements physiques (radiations notamment), par des interventions chirurgicales (intestinales notamment), par des coloscopies ou par des apports nutritionnels. La présente invention se rapporte également à l'utilisation d'un lyophilisat de microbiote fécal d'un sujet donneur susceptible d'être obtenu par le procédé selon l'invention, pour traiter une pathologie choisie parmi les maladies inflammatoires chroniques de l'intestin (MICI), les troubles fonctionnels intestinaux, l'obésité, les maladies métaboliques (diabète de type 2, syndrome métabolique notamment) et les maladies auto-immunes (diabète de type 1, notamment), les allergies, les maladies hépatiques (stéatose, cirrhose notamment), certaines maladies neurologiques (autisme notamment) et certains cancers (cancer colorectal notamment).

Par dysbiose intestinale, on entend tout déséquilibre soutenu du microbiote intestinal. Par déséquilibre soutenu du microbiote intestinal, on entend toute perte de micro-organismes bénéfiques, et/ou toute perte de diversité de micro-organismes, et/ou toute expansion ou développement de micro-organismes agressifs parmi les commensaux (pathobiontes), et/ou toute prolifération de micro-organismes pathogènes (*C*. *difficile* notamment). Toute altération soutenue du microbiote intestinal humain peut en effet engendrer ou accompagner de façon chronique un état pathologique. Notamment, la réduction de la diversité au sein du microbiote est caractéristique des maladies associées à une dysbiose (obésité, maladie de Crohn, diabète ou allergie notamment) (Sansonetti, Collège de France, 22 janvier 2014).

De préférence, la pathologie à traiter est une dysbiose intestinale.

Par maladies inflammatoires chroniques de l'intestin (MICI), on entend notamment la maladie de Crohn, la rectocolite hémorragique.

Par troubles fonctionnels intestinaux, on entend notamment le syndrome du côlon irritable, la colite spasmodique.

Le procédé de préparation d'un lyophilisat de microbiote fécal d'un sujet donneur selon l'invention comprend ainsi les étapes suivantes :
A) le mélange d'un échantillon de microbiote fécal d'un sujet donneur avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A) à une température inférieure à -50°C, de préférence comprise entre -70°C et -100°C, puis sa lyophilisation.

De préférence, l'échantillon de microbiote fécal du sujet donneur est un échantillon de selles dudit donneur. En effet, l'échantillon de selles contient du microbiote fécal du sujet donneur. De préférence, selon l'invention le sujet donneur est un sujet humain sain. Par sujet « sain », on entend un sujet non atteint d'un déséquilibre du microbiote intestinal ou d'une pathologie diagnostiquée/reconnue par le corps médical. De préférence, l'échantillon de microbiote fécal du sujet donneur est préalablement purifié. Alternativement, de préférence, selon l'invention le sujet donneur est un sujet humain malade.

De préférence, l'échantillon de selles présente une masse d'au moins 20g. L'échantillon de microbiote fécal est toujours obtenu et mélangé en anaérobiose (*i.e.* sous une atmosphère dépourvue d'oxygène). En anaérobiose, la viabilité des bactéries constitutives du microbiote fécal et présentes dans l'échantillon est ainsi préservée.

De préférence, préalablement à son utilisation, l'échantillon de microbiote fécal est filtré en anaérobiose. L'étape préalable de filtration peut comprendre, en anaérobiose, la filtration en utilisant un sac Seward muni de filtre.

De préférence, l'échantillon de microbiote fécal est prélevé en anaérobiose, en utilisant un dispositif de collecte étanche à l'air. De préférence, ce dispositif se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps,
dans lequel le corps du conteneur est constitué d'une poche souple, et dans lequel au moins l'un parmi le conteneur et le couvercle est pourvu d'un organe d'évacuation adapté pour évacuer au moins une partie des gaz contenus dans l'espace intérieur du corps du conteneur.

Alternativement, le dispositif de collecte étanche à l'air se présente sous une forme du type comprenant :
- un conteneur comprenant un corps qui comporte un espace intérieur adapté pour recevoir l'échantillon de microbiote fécal du sujet donneur, et un col qui délimite une ouverture d'accès à l'espace intérieur du corps, et
- un couvercle adapté pour être monté de manière amovible et étanche sur le col du conteneur de manière à obturer l'ouverture d'accès du col et à fermer l'espace intérieur du corps,
dans lequel l'espace intérieur du corps du conteneur comprend éventuellement un dispositif chimique neutralisant l'oxygène.

Le diluant peut être choisi parmi les composés suivants :
- les cryoprotectants tels que les di- à pentasaccharides, *i.e.* les disaccharides, les trisaccharides, les quadrisaccharides et les pentasaccharides; ou les polyols, tels que le glycérol, le mannitol, le sorbitol, le propylène glycol ou l'éthylène glycol,
- les agents de charge, tels que les hydrolysats partiels d'amidon, notamment de blé ou de maïs, ou de fécule, comprenant une forte quantité de maltodextrines,
- et leurs mélanges.

De préférence, le diluant est une solution aqueuse saline comprenant au moins un cryoprotectant et/ou un agent de charge. Ainsi, typiquement, la solution aqueuse saline comprend de l'eau et des sels physiologiquement acceptables. Typiquement, les sels sont des sels de calcium, sodium, potassium ou magnésium, avec des ions chlorure, gluconate, acétate ou hydrogénocarbonate.

La solution aqueuse saline peut également optionnellement comprendre au moins un antioxydant. L'antioxydant est notamment choisi parmi l'acide ascorbique et ses sels (ascorbate), les tocophérols (notamment l'a-tocophérol), la cystéine et ses formes salifiées (chlorhydrate notamment) et leurs mélanges.

De préférence, la solution aqueuse saline comprend :
- au moins un sel choisi parmi le chlorure de sodium, le chlorure de calcium, le chlorure de magnésium, le chlorure de potassium, le sodium gluconate et le sodium acétate, et
- optionnellement au moins un antioxydant, de préférence choisi parmi le L-ascorbate de sodium, les tocophérols, le chlorhydrate de L-cystéine monohydraté et leurs mélanges.

Typiquement, le sel est présent dans la solution aqueuse saline en concentration comprise entre 5 et 20g/L, de préférence entre 7 et 10 g/L.

Typiquement, l'antioxydant est présent dans la solution aqueuse saline en quantité comprise entre 0.3 et 1% en poids par rapport au volume total de solution, de préférence en quantité comprise entre 0.4 et 0.6% en poids par rapport au volume total de solution. De préférence, lorsque l'antioxydant est un mélange de L-ascorbate de sodium et de chlorhydrate de L-cystéine monohydraté, le L-ascorbate de sodium est présent en quantité comprise entre 0.4 et 0.6% en poids par rapport au volume total de solution, et le chlorhydrate de L-cystéine monohydraté est présent en quantité comprise entre 0.01 et 0.1% en poids par rapport au volume total de solution.

La solution aqueuse saline comprend au moins un cryoprotectant. Un cryoprotectant est une substance utilisée pour protéger l'échantillon des dommages causés par la congélation, notamment dus à la formation de cristaux de glace.

De préférence, le cryoprotectant est choisi parmi les polyols, les di- à pentasaccharides (*i.e.* les disaccharides, les trisaccharides, les quadrisaccharides et les pentasaccharides), et leurs mélanges. De préférence, le cryoprotectant est choisi parmi les polyols, les tri- et disaccharides et leurs mélanges. Plus préférentiellement, le cryoprotectant présent dans la solution aqueuse saline est un disaccharide ou un trisaccharide.

Parmi les polyols utilisables, on trouve notamment le glycérol, le mannitol, le sorbitol, mais également le propylène glycol ou l'éthylène glycol.

Parmi les di- à pentasaccharides utilisables, on peut citer les dimères, trimères, quadrimères et pentamères d'unités identiques ou différentes, lesdites unités étant choisies parmi le glucose, le fructose, le galactose, le fucose et l'acide N-acétylneuraminique.

Parmi les disaccharides utilisables, on trouve notamment le tréhalose ou l'un de ses analogues, ou le saccharose.

Ces cryoprotectants peuvent être utilisés seuls ou en mélange.

Typiquement, la quantité totale de cryoprotectant présent dans la solution aqueuse saline est comprise entre 3 et 30% en poids par rapport au volume total de solution, de préférence entre 4% et 20% en poids par rapport au volume total de solution.

De préférence, le cryoprotectant est choisi parmi le glycérol, le mannitol, le sorbitol, le propylène glycol, l'éthylène glycol, le tréhalose et ses analogues, le saccharose, le galactose-lactose et leurs mélanges. Plus préférentiellement, le cryoprotectant est le galactose-lactose ou le tréhalose.

La solution aqueuse saline selon l'invention comprend au moins un agent de charge.

L'agent de charge est de préférence choisi parmi les hydrolysats partiels d'amidon ou de fécule. Les hydrolysats partiels d'amidon, notamment de blé ou de maïs, ainsi que les hydrolysats partiels de fécule, par exemple de pomme de terre, comprennent une forte quantité de maltodextrines. Les maltodextrines sont le résultat de l'hydrolyse partielle d'amidon ou de fécule, et sont constituées de différents sucres (glucose, maltose, maltotriose, oligo- et polysaccharides), dont les proportions varient en fonction du degré d'hydrolyse.

De préférence, l'agent de charge présent dans la solution aqueuse saline est un mélange de maltodextrines, dans lequel la quantité de maltodextrines est comprise entre 4 et 20% en poids par rapport au volume total de solution.

De préférence, l'étape A) est réalisée en mélangeant l'échantillon de microbiote fécal avec le diluant, dans un rapport poids de microbiote, de préférence purifié (g) / volume de diluant (mL) compris entre 1 : 1 et 1 : 10.

Puis le mélange obtenu en A) est congelé à une température inférieure à -50°C, de préférence comprise entre -70°C et -100°C, puis soumis à lyophilisation : c'est l'étape B). Elle est réalisée de préférence en conditions suivantes :
B1) la congélation du mélange obtenu en A) à une température inférieure à -50°C, de préférence comprise entre -70°C et -100°C, de préférence à une température d'environ - 80°C,
B2) le chargement du mélange congelé obtenu en B1) dans un lyophilisateur pré-refroidi à une température comprise entre -50°C et -30°C, à pression atmosphérique, puis
B3) au moins une étape de dessiccation primaire du mélange chargé en B2) comprenant l'abaissement de la pression jusqu'à une valeur comprise entre 80 et 200 µbar (préférentiellement entre 100 et 150 µbar) puis l'augmentation de la température des étagères jusqu'à une valeur comprise entre -20°C et +25°C (préférentiellement -10°C) en appliquant une vitesse de chauffage comprise entre 0,2 et 0,5 °C/min. Les valeurs de pression et de température des étagères sont choisies de façon à ce que la température du produit soit maintenue en dessous de la température d'effondrement tout au long de la sublimation. Les paramètres sont maintenus constants jusqu'à l'élimination complète de la glace du mélange, puis
B4) la dessiccation secondaire du mélange obtenu en B3) comprenant l'abaissement de la pression à une valeur inférieure ou égale à 80 µbar, de préférence la valeur minimale possible pour l'équipement, et l'élévation de la température des étagères à une valeur comprise entre +25°C et +35°C, préférentiellement 25°C à une vitesse de chauffage comprise entre 0,1 et 0,3 °C/min, et l'y maintenir entre 8 et 15 heures.

De préférence, la congélation de l'étape B1) est effectuée à une température inférieure à -50°C, de préférence comprise entre -70°C et -100°C. De préférence, la température de congélation est comprise entre -70°C et -100°C ; plus préférentiellement elle est d'environ -80°C ou d'environ -100°C. Afin d'être congelé, le mélange obtenu en A) peut être préalablement aliquoté, pour assurer des spécimens de volume constant.

L'échantillon congelé est ainsi chargé dans un lyophilisateur pré-refroidi à une température comprise entre -50°C et -30°C, à pression atmosphérique ; c'est l'étape B2).

Puis, la dessiccation primaire du mélange chargé en B2) intervient ; c'est l'étape B3). Elle comprend au moins une étape de dessiccation primaire du mélange chargé en B2) comprenant l'abaissement de la pression jusqu'à une valeur comprise entre 80 et 200 µbar (préférentiellement entre 100 et 150 µbar) puis l'augmentation de la température des étagères jusqu'à une valeur comprise entre -20°C et +25°C (préférentiellement - 10°C) en appliquant une vitesse de chauffage comprise entre 0,2 et 0,5 °C/min. Les valeurs de pression et de température étagères sont choisies de façon à ce que la température du produit soit maintenue en dessous de la température d'effondrement tout au long de la sublimation. Les paramètres sont maintenus constants jusqu'à l'élimination complète de la glace du mélange.

Enfin, B4) la dessiccation secondaire du mélange obtenu en B3) est effectuée. Elle comprend l'abaissement de la pression à une valeur inférieure ou égale à 80 µbar, de préférence la valeur minimale possible pour l'équipement, et l'élévation de la température des étagères à une valeur comprise entre +25°C et +35°C, préférentiellement 25°C à une vitesse de chauffage comprise entre 0,1 et 0,3 °C/min, et l'y maintenir entre 8 et 15 heures.

On obtient ainsi *in fine* un lyophilisat selon l'invention.

De préférence, le procédé selon l'invention comprend, en étape A), des étapes préalables de traitement de l'échantillon de microbiote fécal, avant de le mélanger au diluant.

Ainsi, de préférence, l'étape A) comprend les sous-étapes suivantes :
A1) optionnellement, la préparation d'un gradient continu d'iodixanol ou de 5- (N-2, 3-dihydroxypropylacetamido)-2, 4, 6-tri-iodo-N, N'-bis (2, 3 dihydroxypropyl) isophthalamide formé par congélation-décongélation,
A2) le mélange d'au moins un échantillon de microbiote fécal d'un sujet donneur avec un tampon salin comprenant éventuellement de l'iodixanol ou du 5- (N-2, 3-dihydroxypropylacetamido)-2, 4, 6-tri-iodo-N, N'-bis (2, 3 dihydroxypropyl) isophthalamide, en anaérobiose,
A3) optionnellement, le dépôt du mélange obtenu en A2) sous le gradient obtenu en A1),
A4) la centrifugation séquentielle à faible accélération en anaérobiose, ou l'ultracentrifugation du mélange obtenu en A2) ou A3),
A5) la récupération de l'anneau bactérien ou du surnageant formé à l'issue de l'étape A4), en anaérobiose, et
A6) le mélange de l'anneau bactérien ou du surnageant récupéré en A5) avec un diluant qui est une solution aqueuse saline comprenant (i) au moins un cryoprotectant choisi parmi les polyols, les di- à pentasaccharides, ou leurs mélanges, et (ii) des maltodextrines.

Dans un tel procédé, l'étape A1), ainsi que *de facto* l'étape A3), sont facultatives.

De préférence, le tampon salin utilisé dans les différentes sous-étapes décrites ci-dessus est une solution aqueuse d'HEPES comprenant du chlorure de sodium, de préférence à une concentration comprise entre 7 et 15g/1. De préférence, l'HEPES est présent en concentration comprise entre 8 et 50 mM, de préférence entre 9 et 42 mM.

### Première alternative

De préférence, selon une première alternative, le procédé selon l'invention comprend les étapes suivantes :
A1) la préparation d'un gradient continu d'iodixanol ou de 5- (N-2, 3-dihydroxypropylacetamido)-2, 4, 6-tri-iodo-N, N'-bis (2, 3 dihydroxypropyl) isophthalamide formé par congélation-décongélation,
A2) le mélange d'au moins un échantillon de microbiote fécal d'un sujet donneur avec un tampon salin additionné d'une solution aqueuse comprenant de l'iodixanol ou du 5-(N-2, 3-dihydroxypropylacetamido)-2, 4, 6-tri-iodo-N, N'-bis (2, 3 dihydroxypropyl) isophthalamide, ledit tampon salin étant de préférence une solution aqueuse d'HEPES comprenant du chlorure de sodium, en anaérobiose,
A3) le dépôt du mélange obtenu en A2) sous le gradient obtenu en A1),
A4) l'ultracentrifugation du mélange obtenu en A3), pendant une durée comprise entre 40 et 50 minutes, à une température comprise entre 2°C et 6°C, à une accélération comprise entre 13000 et 16000 x g,
A5) la récupération de l'anneau bactérien formé à l'issue de l'étape A4), en anaérobiose,
A6) le mélange de l'anneau bactérien récupéré en A5) avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A6) à une température inférieure à -50°C, de préférence comprise entre -70°C et -100°C, puis sa lyophilisation.

L'étape A1) comprend ainsi la préparation d'un gradient continu d'iodixanol ou de 5-(N-2, 3-dihydroxypropylacetamido)-2, 4, 6-tri-iodo-N, N'-bis (2, 3 dihydroxypropyl) isophthalamide formé par congélation-décongélation.

Par gradient continu d'iodixanol, on entend un gradient continu d'iodixanol de densité allant de 1.03 à 1.24, de préférence allant de 1.06 à 1.24.

Par gradient continu de 5- (N-2, 3-dihydroxypropylacetamido)-2, 4, 6-tri-iodo-N, N'-bis (2, 3 dihydroxypropyl) isophthalamide, on entend un gradient continu de 5- (N-2, 3-dihydroxypropylacetamido)-2, 4, 6-tri-iodo-N, N'-bis (2, 3 dihydroxypropyl) isophthalamide de densité allant de 1,03 à 1,22.

De préférence, cette étape A1) est réalisée selon les étapes suivantes :
A1.a) congélation d'une solution d'iodixanol ou de 5- (N-2, 3-dihydroxypropylacetamido)-2, 4, 6-tri-iodo-N, N'-bis (2, 3 dihydroxypropyl) isophthalamide à une température comprise entre -70°C et -100°C pendant au moins 12 heures. De préférence, ladite solution est dégazée. Par solution d'iodixanol ou de 5- (N-2, 3-dihydroxypropylacetamido)-2, 4, 6-tri-iodo-N, N'-bis (2, 3 dihydroxypropyl) isophthalamide dégazée, on entend une solution d'iodixanol ou de 5- (N-2, 3-dihydroxypropylacetamido)-2, 4, 6-tri-iodo-N, N'-bis (2, 3 dihydroxypropyl) isophthalamide dans laquelle la concentration en air dissous est réduite, par exemple sous vide. Cela permet de créer une micro-anaérobiose favorable à la survie des bactéries au cours du procédé de purification ; puis
A1.b) décongélation de la solution obtenue en A1.a) à température ambiante pendant 2 à 4 heures, afin d'obtenir un gradient continu d'iodixanol ou de 5- (N-2, 3-dihydroxypropylacetamido)-2, 4, 6-tri-iodo-N, N'-bis (2, 3 dihydroxypropyl) isophthalamide.

De préférence, la solution d'iodixanol utilisée à l'étape A1) est une solution présentant une concentration comprise entre 15 et 25%, préférentiellement aux alentours de 20%, en poids d'iodixanol par volume de solution (p/v). Plus préférentiellement, la solution d'iodixanol utilisée est obtenue en diluant 3 fois une solution aqueuse commerciale d'iodixanol à 60%, commercialisée sous le nom OptiPrep (solution aqueuse stérile à 60% p/v d'iodixanol), dans un tampon salin, préférentiellement un tampon comprenant de l'HEPES 15mM et du NaCl 9 g/L ayant un pH de 7.0.

De préférence, la solution de 5- (N-2, 3-dihydroxypropylacetamido)-2, 4, 6-tri-iodo-N, N'-bis (2, 3 dihydroxypropyl) isophthalamide est une solution comprenant 20% en poids de 5- (N-2, 3-dihydroxypropylacetamido)-2, 4, 6-tri-iodo-N, N'-bis (2, 3 dihydroxypropyl) isophthalamide par volume de solution (p/v). Plus préférentiellement, le 5- (N-2, 3-dihydroxypropylacetamido)-2, 4, 6-tri-iodo-N, N'-bis (2, 3 dihydroxypropyl) isophthalamide utilisé est commercialisé par Progen Biotechnik sous le nom Nycodenz.

De préférence, dans l'étape A1.a), la congélation de la solution d'iodixanol ou de 5- (N-2, 3-dihydroxypropylacetamido)-2, 4, 6-tri-iodo-N, N'-bis (2, 3 dihydroxypropyl) isophthalamide peut être effectuée pendant plusieurs jours, voire 1 mois.

Une fois le gradient continu d'iodixanol ou de 5- (N-2, 3-dihydroxypropylacetamido)-2, 4, 6-tri-iodo-N, N'-bis (2, 3 dihydroxypropyl) isophthalamide obtenu à l'étape A1), les étapes ultérieures A2), A4) et A5) sont réalisées en anaérobiose.

L'étape A2) comprend le mélange d'au moins un échantillon de microbiote fécal d'un sujet donneur avec un tampon salin additionné d'une solution aqueuse comprenant de l'iodixanol ou du 5- (N-2, 3-dihydroxypropylacetamido)-2, 4, 6-tri-iodo-N, N'-bis (2, 3 dihydroxypropyl) isophthalamide, en anaérobiose. De préférence, ledit tampon salin est une solution aqueuse d'HEPES comprenant du chlorure de sodium. De préférence, l'HEPES est présent en concentration comprise entre 30 et 50 mM dans le tampon salin. De préférence, le chlorure de sodium est présent à une concentration comprise entre 7 et 15g/l dans le tampon salin. De préférence, la solution aqueuse d'iodixanol est à une concentration comprise entre 50% et 70%, de préférence à environ 60% (p/v). De préférence, le ratio tampon salin : solution aqueuse d'iodixanol est d'environ 1 : 3.

De préférence, la solution aqueuse de 5- (N-2, 3-dihydroxypropylacetamido)-2, 4, 6-tri-iodo-N, N'-bis (2, 3 dihydroxypropyl) isophthalamide est à une concentration de 60% (p/v). De préférence, le ratio tampon salin : solution aqueuse de 5- (N-2, 3-dihydroxypropylacetamido)-2, 4, 6-tri-iodo-N, N'-bis (2, 3 dihydroxypropyl) isophthalamide est d'environ 1 : 3.

Ainsi, selon l'étape A2), le tampon salin est préalablement mélangé avec une solution aqueuse d'iodixanol ou de 5- (N-2, 3-dihydroxypropylacetamido)-2, 4, 6-tri-iodo-N, N'-bis (2, 3 dihydroxypropyl) isophthalamide ; on obtient ainsi un tampon salin comprenant de l'iodixanol ou de 5- (N-2, 3-dihydroxypropylacetamido)-2, 4, 6-tri-iodo-N, N'-bis (2, 3 dihydroxypropyl) isophthalamide. Puis le mélange résultant est mélangé avec l'échantillon de microbiote fécal.

De préférence, le mélange de l'échantillon de microbiote fécal avec un tampon salin comprenant de l'iodixanol ou du 5- (N-2, 3-dihydroxypropylacetamido)-2, 4, 6-tri-iodo-N, N'-bis (2, 3 dihydroxypropyl) isophthalamide s'effectue dans un ratio de 3 à 4 grammes d'échantillon pour 22 à 30ml de tampon salin comprenant de l'iodixanol ou du 5- (N-2, 3-dihydroxypropylacetamido)-2, 4, 6-tri-iodo-N, N'-bis (2, 3 dihydroxypropyl) isophthalamide.

De préférence, lorsque l'échantillon de microbiote fécal est filtré entre les étapes A2) et A3), il subit une étape de filtration du mélange obtenu en A2) en anaérobiose, notamment en utilisant un sac Seward muni de filtre.

Ensuite, le mélange obtenu en A2), éventuellement filtré, est déposé sous le gradient obtenu en A1) : c'est l'étape A3). L'étape A3) peut être effectuée en aérobiose ou en anaérobiose.

De préférence, le mélange obtenu en A2) est présent dans une seringue munie d'une aiguille, et le gradient d'iodixanol ou de 5- (N-2, 3-dihydroxypropylacetamido)-2, 4, 6-tri-iodo-N, N'-bis (2, 3 dihydroxypropyl) isophthalamide est présent dans un récipient de type tube. Dans ce cas, le mélange obtenu en A2) est déposé en plongeant l'aiguille de la seringue au fond du tube contenant le gradient d'iodixanol ou de 5- (N-2, 3-dihydroxypropylacetamido)-2, 4, 6-tri-iodo-N, N'-bis (2, 3 dihydroxypropyl) isophthalamide, et en vidant le contenu de la seringue.

Puis, la préparation obtenue en A3) est centrifugée sous vide dans une ultracentrifugeuse à faible accélération: c'est l'étape A4). Cette ultracentrifugation est effectuée pendant une durée comprise entre 40 et 50 minutes, à une température comprise entre 2°C et 6°C, à une vitesse comprise entre 13000 et 16000 x g. De préférence, l'ultracentrifugation est effectuée pendant une durée comprise entre 40 et 50 minutes, à une température d'environ 4°C, à une vitesse d'environ 14500-14600 x g.

A l'issue de cette étape, un anneau bactérien s'est formé au sein du gradient. Cet anneau est récupéré en anaérobiose dans l'étape A5). Il contient le microbiote d'intérêt.

### Seconde alternative

De préférence, selon une seconde alternative, le procédé de préparation comprend les étapes suivantes :
A2) le mélange d'au moins un échantillon de microbiote fécal d'un sujet donneur avec un tampon salin, en anaérobiose,
A4) la centrifugation séquentielle à faible accélération du mélange obtenu en A2), en anaérobiose,
A5) la récupération du surnageant formé à l'issue de l'étape A4), en anaérobiose,
A6) le mélange du surnageant récupéré en A5) avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A6) à une température inférieure à -50°C, de préférence comprise entre -70°C et -100°C, puis sa lyophilisation.

Cette seconde alternative comprend une étape de centrifugation séquentielle à faible accélération. Par faible accélération, on entend une accélération comprise entre 200 et 500 x g. Ainsi, les différentes particules présentes dans l'échantillon de microbiote fécal (débris puis cellules bactériennes) vont sédimenter.

L'étape A2) comprend le mélange d'au moins un échantillon de microbiote fécal d'un sujet donneur avec un tampon salin, en anaérobiose. De préférence, ledit tampon salin est une solution aqueuse d'HEPES comprenant du chlorure de sodium. De préférence, l'HEPES est présent en concentration comprise entre 5 et 15 mM. De préférence, le chlorure de sodium est présent à une concentration comprise entre 7 et 15g/l.

De préférence, le mélange de l'échantillon de microbiote fécal avec un tampon salin s'effectue dans un ratio pondéral échantillon : tampon de 1 :20 à 1 : 25.

De préférence, lorsque l'échantillon de microbiote fécal est filtré entre les étapes A2) et A4), il subit une étape de filtration du mélange obtenu en A2) en anaérobiose, notamment en utilisant un sac Seward muni de filtre.

Ensuite, le mélange obtenu en A2), éventuellement filtré, est soumis à une centrifugation séquentielle à faible accélération, en anaérobiose : c'est l'étape A4).

La centrifugation séquentielle à faible accélération de l'étape A4) est de préférence réalisée à une accélération comprise entre 200 et 500 x g pendant un temps compris entre 5 et 15 minutes, à une température comprise entre 20 et 30°C. Plus préférentiellement, la centrifugation séquentielle à faible accélération est réalisée à une accélération d'environ 300 x g pendant un temps compris entre 5 et 15 minutes, à une température comprise entre 20 et 25°C.

A l'issue de cette étape, le surnageant est récupéré en anaérobiose (étape A5)). Il contient le microbiote d'intérêt.

De préférence, la centrifugation séquentielle à faible accélération de l'étape d) et l'étape de récupération du surnageant subséquente e) sont réalisées plusieurs fois, de préférence au moins 2 fois, chaque étape d) étant réalisée à une accélération comprise entre 200 et 500 x g pendant un temps compris entre 5 et 15 minutes et à une température comprise entre 20 et 30°C.

Ainsi, de préférence, dans l'étape A5) :
A5.1) le surnageant formé à l'issue de l'étape A4) est récupéré,
A5.2) le culot obtenu en A4) est mélangé avec un tampon salin (le même que celui de l'étape A2)), puis soumis à centrifugation séquentielle à faible accélération comme décrit à l'étape A4), et le surnageant ainsi obtenu est mélangé avec le surnageant de la fraction A5.1). Cette opération de lavage du culot peut être répétée à volonté jusqu'à épuisement du culot en bactéries. Dans la pratique et par mesure d'économie de temps et de volumes d'extraction, un seul lavage du culot est typiquement pratiqué.

Quel que soit le procédé selon l'invention (première ou seconde alternative), ce dernier peut comprendre les étapes suivantes, en anaérobiose, en tant qu'étape A6) :
a) centrifugation de l'anneau bactérien remis en suspension dans un tampon salin ou du surnageant obtenu en A5), à une accélération comprise entre 3000 et 4000 x g pendant un temps compris entre 5 et 15 minutes, à une température comprise entre 20 et 30°C,
b) récupération du culot obtenu à l'issue de l'étape a), et remise en suspension dans un tampon salin, puis centrifugation à une accélération comprise entre 200 et 500 x g pendant un temps compris entre 5 et 15 minutes, à une température comprise entre 20 et 30°C,
c) récupération du surnageant obtenu à l'issue de l'étape b), et remise en suspension dans un tampon salin, puis centrifugation à une accélération comprise entre 3000 et 4000 x g pendant un temps compris entre 5 et 15 minutes, à une température comprise entre 20 et 30°C,
d) récupération du culot obtenu à l'issue de l'étape c), et
e) le mélange du culot récupéré en d) avec un diluant qui est une solution aqueuse saline comprenant (i) au moins un cryoprotectant choisi parmi les polyols, les di- à pentasaccharides, ou leurs mélanges, et (ii) des maltodextrines. Ces étapes a) à e) peuvent ainsi être des sous-étapes de l'étape A6). Les étapes a) à d) visent à laver le microbiote obtenu en A5). De préférence, la température des étapes a) à d) est comprise entre 20 et 25°C.

De préférence, dans ces étapes a) à d), le tampon salin est une solution aqueuse d'HEPES comprenant du chlorure de sodium, de préférence à une concentration comprise entre 7 et 15g/l. De préférence, l'HEPES est présent en concentration comprise entre 8 et 15 mM.

Le culot obtenu à l'étape d) contient le microbiote d'intérêt. Il peut ainsi être mélangé au diluant, comme décrit dans l'étape e).

A l'issue de l'étape A5) ou d) (peu importe les procédés selon la première ou seconde alternative), la fraction récupérée est mélangée avec un diluant qui est une solution aqueuse saline comprenant (i) au moins un cryoprotectant choisi parmi les polyols, les di- à pentasaccharides, ou leurs mélanges, et (ii) des maltodextrines.

Puis, l'étape B) de congélation et lyophilisation se déroule, comme décrit ci-avant.

La présente invention se rapporte également à l'utilisation d'un lyophilisat susceptible d'être obtenu par le procédé selon l'invention, comme outil de recherche, notamment comme décrit ci-avant, en génomique fonctionnelle, en métaprotéomique ou en immunologie.

La présente invention se rapporte également à un lyophilisat de microbiote fécal d'un sujet donneur susceptible d'être obtenu par le procédé selon l'invention, pour son utilisation dans la transplantation de microbiote fécal autologue ou allogénique. En effet, le lyophilisat de microbiote fécal purifié selon le procédé de l'invention peut être administré au patient receveur.

Le patient receveur peut être différent du sujet donneur, et la transplantation est alors allogénique.

Le patient receveur peut aussi être identique au sujet donneur, et la transplantation est alors autologue ; ce type de transplantation peut avoir lieu lorsque le sujet, alors sain, donne un échantillon avant l'altération de son microbiote. Le lyophilisat est alors conservé, puis transplanté à ce même sujet (patient receveur) si celui-ci présente notamment une infection à *Clostridium difficile.* La transplantation de microbiote fécal autologue présente l'avantage d'éviter la transmission d'agent pathogène provenant d'un autre donneur.

La présente invention se rapporte également à un lyophilisat de microbiote fécal d'un sujet donneur susceptible d'être obtenu par le procédé selon l'invention, pour son utilisation pour traiter les infections à *Clostridium difficile.* La présente invention se rapporte également à un lyophilisat de microbiote fécal d'un sujet donneur susceptible d'être obtenu par le procédé selon l'invention, pour son utilisation pour accompagner le traitement ou pour traiter une pathologie choisie parmi les maladies inflammatoires chroniques de l'intestin (MICI), les troubles fonctionnels intestinaux, l'obésité, les maladies métaboliques et les maladies auto-immunes, les allergies, les maladies neurologiques et les cancers. La présente invention se rapporte également à un lyophilisat susceptible d'être obtenu par le procédé selon l'invention, pour son utilisation pour limiter les effets secondaires d'un traitement choisi parmi les antibiothérapies, les chimiothérapies, les radiothérapies et les chirurgies notamment de l'appareil digestif.

Le lyophilisat susceptible d'être obtenu par le procédé selon l'invention présente une bonne viabilité des bactéries présentes, comme démontré en exemple 4.

Typiquement, la viabilité des bactéries du microbiote fécal est mesurée grâce au marquage LIVE/DEAD® *Bac*Light™ Bacterial Viability Kit commercialisé par ThermoFisher Scientific. Ce kit permet en effet de distinguer les bactéries vivantes et mortes sur la base de l'intégrité de leurs membranes, par l'intermédiaire de deux fluorophores, le SYTO9® et l'iodure de propidium (PI). Le premier pénètre dans toutes les cellules, intègres ou non, se fixe à l'ADN et émet à 540nm (vert) après excitation à 470nm (laser bleu). Le PI cible également l'ADN, mais pénètre uniquement dans les cellules dont les membranes sont endommagées ; il émet à 635nm (rouge) après excitation à 470nm. Un tel kit peut être associé à la cytométrie en flux ou la microscopie en épifluorescence.

De préférence, le marquage des bactéries avec le mélange des deux fluorophores SYTO9®/PI, est réalisé en anaérobiose.

L'invention va maintenant être exemplifiée à l'aide des exemples qui suivent, qui ne sont pas limitatifs.

### Description des figures :

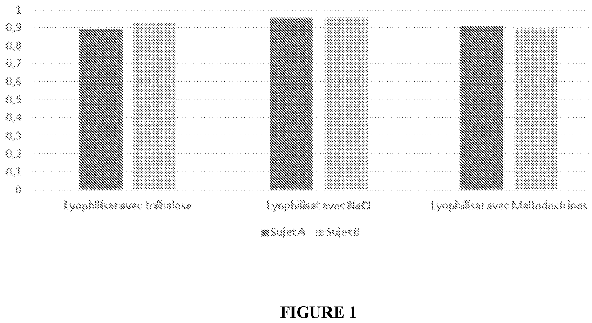

La figure 1 est un diagramme qui illustre les corrélations de Pearson entre lyophilisats et les selles brutes.

### Exemple 1: Purification d'un échantillon de microbiote fécal d'un sujet donneur par gradient continu d'iodixanol (OptiPrep) selon l'invention

### Principe :

Séparation de la fraction bactérienne totale, par flottaison au sein d'un gradient continu d'OptiPrep auto-formé par congélation-décongélation. La dilution fécale alourdie en OptiPrep est déposée sous un gradient continu d'OptiPrep, préformé par congélation-décongélation. Au cours de la centrifugation, les bactéries remontent au sein du gradient jusqu'à leur densité de flottaison (1.110-1.190), alors que les débris alimentaires et endogènes plongent au fond du gradient. Tout le procédé est en anérobiose.

### Matériels & Méthodes:

### Tampons Hepes-NaCl

| | NaCl | Hepes | QSP MilliQ |
|---|---|---|---|
| ***Pour diluant pour suspensions fécales*** | 0,9 g | 953,2 mg | QSP < 100 mL |
| Hepes 40mM-NaCl 9g/L | | | puis ajuster pH 7.0 |
| | | | puis QSP 100 mL |
| Le diluant pour suspensions fécales comprend *in fine,* pour 100ml : - 25mL d'Hepes 40mM-NaCl 9g/L et - 75mL d'OptiPrep-60 (solution commerciale à 60% d'iodixanol dans l'eau) | | | |

| ***Pour gradients*** | | | |
|---|---|---|---|
| Hepes 15mM-NaCl 9g/L | 0,9 g | 357,5 mg | QSP < 100 mL |
| | | | puis ajuster pH 7.0 |
| Le diluant pour gradients (« OptiPrep-20 ») est *in fine* une dilution de 3 fois OptiPrep-60 dans Hepes 15mM-NaCl 9g/L | | | puis QSP 100 mL |

| ***Pour lavages phase bactérienne*** | | | |
|---|---|---|---|
| Hepes 10mM-NaCl 9g/L | 18,0 g | 4,766 g | QSP < 2 L |
| | | | puis ajuster pH 7.0 |
| | | | puis QSP 2 L |

### Préparation des gradients continus par congélation-décongélation (étape A1)) :

- pipeter 16mL de solution OptiPrep-20 dégazée, et transférer en tube en évitant d'aérer la solution
- congeler les tubes overnight à -80°C
- décongeler les tubes immobiles et sans perturbation à température ambiante 2-3 heures avant utilisation; un gradient continu de densité 1.03-1.22 se forme automatiquement.

### Préparation des dilutions fécales en enceinte anaérobie (étape A2)) :

- Rentrer l'échantillon de selles dans l'enceinte anaérobie
- Peser sur la balance, sur laquelle est placé un sachet stérile Seward à double filtre, le poids de selles souhaité (maximum 3,5g pour 1 gradient, possiblement moins ; dans ce cas compléter à 3,5g avec tampon Hepes 10mM-NaCl 9g/L)
- Ajouter dans le filtre du sac Seward, 24mL de diluant pour suspensions fécales
- Homogénéiser le mélange
- transférer la suspension fécale homogénéisée filtrée dans un tube type Falcon 50mL
- A partir de ce tube, remplir une seringue de 20mL équipée d'une aiguille avec la dilution fécale

### Réalisation du gradient à l'extérieur de l'enceinte anaérobie (étapes A3) et A4)) :

- Plonger l'aiguille de la seringue obtenue en A2) au fond du gradient préformé par congélation-décongélation obtenu en A1)
- Charger doucement les 20mL de dilution fécale sous le gradient préformé
- Recommencer pour les autres gradients
- Peser tous les tubes et ajuster exactement au même poids les tubes 2 à 2 avec tampon Hepes 10mM-NaCl 9g/L
- Insérer délicatement les tubes dans les plots de centrifugation froids pour rotor oscillant
- Centrifuger 45 min à 4°C 14,567 × *g*

### Récupération et lavage des cellules bactériennes en enceinte anaérobie (étapes A5), A6) et a) à d)) :

- Entrer en chambre anaérobie les plots de centrifugation sans les ouvrir
- Ouvrir un plot
- A l'aide d'une pipette, éliminer la phase supérieure
- Pipeter la phase cellulaire intermédiaire et la répartir dans 2 tubes Falcon de 50mL
- Ajouter du tampon de lavage (Hepes 10mM-NaCl 9g/L) jusqu'à la graduation 50 des 2 tubes Falcon
- Prélever de la même manière les phases bactériennes de tous les gradients
- Centrifuger 10 min 4000 × *g* 22°C en rotor oscillant
- Eliminer le surnageant par aspiration
- Ajouter du tampon de lavage à la graduation ∼ 25mL des tubes Falcon
- Remettre les bactéries doucement en suspension à l'aide d'une pipette ; compléter à 50mL
- Centrifuger 5min 300 × *g* 22°C en rotor oscillant pour éliminer les débris résiduels
- Transférer avec une pipette le surnageant (contenant les bactéries sans résidu) dans 2 nouveaux Falcons 50mL ; jeter le culot de débris
- Centrifuger 10 min 3500 × *g* 22°C en rotor oscillant
- Eliminer les surnageants

Le culot bactérien sans résidu obtenu peut être remis en suspension dans le véhicule choisi.

### Exemple 2: Purification d'un échantillon de microbiote fécal d'un sujet donneur par centrifugation séquentielle à faible accélération selon l'invention

### Principe :

Séparation de la fraction bactérienne totale, par centrifugation séquentielle à faible accélération.

### Matériels & Méthodes:

### Tampon Hepes-NaCl

| | NaCl | Hepes | QSP MilliQ |
|---|---|---|---|
| ***pour suspensions fécales et lavages phases bactériennes*** | | | QSP < 2 L |
| Hepes 10mM-NaCl 9G/L | 18,0 g | 4,766 g | puis ajuster pH 7.0 |
| | | | puis QSP 2 L |

### Préparation des dilutions fécales en enceinte anaérobie:

- Rentrer l'échantillon de selles dans l'enceinte anaérobie
- Transférer le poids de selles désiré dans le filtre du sachet Seward
- Compléter QSP 350g avec Hepes 10mM-NaCl 9g/L pour 14g de selles (soit une dilution 1 :25) et homogénéiser (étape b))
- transférer 50mL de suspension fécale homogénéisée filtrée dans 6 tubes type Falcon 50mL
- Centrifuger 10 min 300 × *g* 22°C en rotor oscillant pour éliminer les débris (étape d))
- Répartir les surnageants (ils contiennent les bactéries) dans 12 nouveaux tubes 50mL (∼25mL / tube)
- Remettre les 6 culots en suspension dans 50mL Hepes 10mM-NaCl 9g/L et centrifuger 10 min 300 × *g* 22°C en rotor oscillant (étape d))
- Répartir les 6 nouveaux surnageants dans les 12 tubes en attente contenant déjà les premiers surnageants ; QSP 50mL pour les 12 tubes avec Hepes 10mM-NaCl 9g/L
- Centrifuger les 12 tubes 10 min 3500 × *g* 22°C en rotor oscillant pour culoter les bactéries
- A l'aide d'une pipette, éliminer délicatement le surnageant

Au final, les culots bactériens sans résidu obtenus sont remis en suspension dans le véhicule choisi.

### Exemple 3: Obtention des lyophilisats de microbiote fécal obtenus aux exemples 1 et 2

Les fractions obtenues à la fin des procédés des exemples 1 et 2 ont été mélangées aux diluants suivants :
- NaCl : 9g/L
- Maltodextrines : tréhalose 15/5 ou 5/15 dans NaCl 9g/L1
- Iodixanol : solution aqueuse commerciale à 60% diluée 3 fois en tampon HEPES 40mM-NaCl 9g/L

Puis elles ont été congelées à -80°C ou -100°C

Puis elles ont été soumises au cycle de lyophilisation suivant :

| **Etapes** | **Température étagères** | **Rampe en min** | **Pression µbar** | **Palier en min** |
|---|---|---|---|---|
| Pré-refroidissement | - 40°C | 60 | atmo | Jusqu'au chargement |
| Dessiccation laire | -10°C | 360 | 150 | 1080 |
| Dessiccation laire | +25°C | 120 | 150 | 720 |

La dessiccation secondaire est effectuée à +25°C, à une pression de 80 µbar pendant 900 minutes environ.

Le lyophilisateur a été pré-refroidi à -40°C avant le chargement. Dès réception des échantillons, les flacons stockés dans la carboglace ont été chargés dans l'appareil et le lyophilisateur a été mis sous vide dès la fin du chargement. Deux sondes de température PT100 ont été placées dans deux flacons. Les produits étant congelés avant chargement, les sondes sont au-dessus du produit et non dans le produit.

La durée du cycle est de 45 h. A la fin du cycle, les flacons sont bouchés sous vide dans le lyophilisateur puis capsulés après déchargement.

### Exemple 4: Viabilité des bactéries présentes dans les lyophilisats de microbiote fécal obtenus à l'exemple 3

### Protocole :

La viabilité des bactéries présentes dans les lyophilisats de microbiote fécal obtenus à l'exemple 3 a été mesurée selon le protocole suivant :
- dilutions décimales successives en atmosphère anaérobie, pour se situer au final autour de 10⁶ bactéries/mL dans l'échantillon destiné au marquage ; cette opération doit être immédiatement consécutive à la remise en suspension des culots bactériens sans résidu dans le véhicule choisi; le marquage de la dernière dilution doit également être immédiat,
- Compter moins de 30min entre la remise en suspension et le marquage, car les populations vivantes prolifèrent rapidement si le véhicule contient un substrat nutritif : en 4 heures d'attente à température ambiante, en anaérobiose et en présence d'un seul substrat nutritif, la population croit d'un demi log et le pourcentage de bactéries vivantes augmente de 10%,
- marquage des bactéries, en utilisant le LIVE/DEAD® *Bac*Light™ Bacterial Viability Kit selon les instructions du fabricant,
- quantification des bactéries vivantes et mortes en cytométrie en flux : le temps entre le marquage et la quantification ne dépasse pas 20 minutes, que les échantillons marqués attendent (toujours à l'abri de la lumière) à température ambiante ou sur glace pilée.

### Résultats :

Les résultats figurent dans le tableau ci-dessous :

| **Echantillon** | **Viabilité 1 semaine après lyophilisation** | **Ecart-type** | **Viabilité 1 mois après lyophilisation** | **Ecart-type** | **Viabilité 3 mois après lyophilisation** | **Ecart-type** |
|---|---|---|---|---|---|---|
| **Lyophilisat avec NaCl** | 8,50% | 2,50% | 5,70% | 3,00% | 12,10% | 3,10% |
| **Lyophilisat avec maltodextrines s** | 44,80% | 8,00% | 48,10% | 5,80% | 50,20% | 2,20% |
| **Lyophilisat avec tréhalose** | 31,10% | 6,80% | 37,60% | 6,90% | 40,30% | 9,80% |
| **Lyophilisat avec iodixanol** | 45,30% | 6,60% | 32,50% | 15,40% | 22,20% | 9,70% |

Ainsi, les résultats montrent que la viabilité des bactéries présentes dans les échantillons purifiés selon l'invention est très bonne lorsque les bactéries sont lyophilisées avec maltodextrines.

### Exemple 5: Obtention et viabilité de bactéries présentes dans des lyophilisats de microbiote fécal non purifiés

Un microbiote a été collecté et mis en suspension dans les solutions suivantes :
- NaCl : 9g/L
- Maltodextrines : tréhalose 15/5 ou 5/15 dans NaCl 9g/L1

Puis les suspensions ont été congelées à -80°C ou -100°C

Puis elles ont été soumises au cycle de lyophilisation suivant :

| **Etapes** | **Température étagères** | **Rampe en min** | **Pression µbar** | **Palier en min** |
|---|---|---|---|---|
| Pré-refroidissement | - 45°C | 60 | atmo | Jusqu'au chargement |
| Dessiccation Iaire | -10°C | 360 | 150 | 1080 |
| Dessiccation Iaire | +25°C | 60 | 150 | 480 |
| Dessiccation Ilaire | +25°C | 1 | 80 | 960 |

Le lyophilisateur a été pré-refroidi à -45°C avant le chargement. Dès réception des échantillons, les produits stockés dans la carboglace ont été chargés dans l'appareil et le lyophilisateur a été mis sous vide dès la fin du chargement.

La durée du cycle est de 48 h. A la fin du cycle, les flacons sont bouchés sous vide dans le lyophilisateur puis capsulés après déchargement.

La qualité des populations bactériennes a été évaluée en termes de diversité via une extraction d'ADN et son analyse par séquençage du gène rDNA 16S. Une analyse phylogénétique a ensuite été menée afin d'établir les profils des différents échantillons afin de les comparer. Les résultats sont présentés dans la Figure 1.

Ainsi, les résultats montrent que les niveaux de corrélation entre les profils taxonomiques sont très élevés pour les 3 formulations, démontrant que le procédé conserve efficacement environ 90% des populations bactériennes.

La viabilité a été évaluée de la même manière que présentée dans l'exemple 4 et les résultats après 10 mois de conservation sont présentés ci-dessous :

| | **Sujet A** | **Sujet B** |
|---|---|---|
| **Lyophilisat avec tréhalose** | 24,90% | 31,20% |
| **Lyophilisat avec NaCl** | 11,30% | 20,20% |
| **Lyophilisat avec Maltodextrines** | 33,80% | 30,70% |

Après 10 mois, les suspensions réalisées avec du NaCl présentent des viabilités nettement plus faibles que les 2 autres.

Ainsi, les microbiotes obtenus selon le procédé permettent à la fois de conserver les populations bactériennes puisque les corrélations des profils phylogénétiques sont très élevées, mais ils permettent de plus de conserver les bactéries en vie plus longtemps que la formulation en NaCl.

## Revendications

1. Procédé de préparation d'un lyophilisat de microbiote fécal d'un sujet donneur, comprenant les étapes suivantes :
A) le mélange d'un échantillon de microbiote fécal d'un sujet donneur avec un diluant qui est une solution aqueuse saline comprenant (i) au moins un cryoprotectant choisi parmi les polyols, les di- à pentasaccharides, ou leurs mélanges, et (ii) des maltodextrines, et
B) la congélation du mélange obtenu en A) à une température inférieure à -50°C, de préférence comprise entre -70°C et -100°C, puis sa lyophilisation.

2. Procédé de préparation d'un lyophilisat selon la revendication 1, **caractérisé en ce que** le diluant est une solution aqueuse saline comprenant au moins le galactose-lactose ou le tréhalose en tant que cryoprotectant.

3. Procédé de préparation d'un lyophilisat selon l'une des revendications 1 ou 2, **caractérisé en ce que** le diluant est une solution aqueuse saline comprenant au moins un mélange de maltodextrines, de préférence dans une quantité comprise entre 4 et 20% par rapport au volume total de la solution.

4. Procédé de préparation d'un lyophilisat selon l'une des revendications 1 à 3, **caractérisé en ce que** la quantité totale de cryoprotectant dans la solution aqueuse saline est comprise entre 3 et 30% en poids par rapport au volume total de la solution, de préférence entre 4 et 20% en poids par rapport au volume totale de la solution.

5. Procédé selon l'une des revendications 1 à 4, **caractérisé en ce que** l'étape A) comprend les étapes suivantes :
A1) optionnellement, la préparation d'un gradient continu d'iodixanol ou de 5- (N-2, 3-dihydroxypropylacetamido)-2, 4, 6-tri-iodo-N, N'-bis (2, 3 dihydroxypropyl) isophthalamide formé par congélation-décongélation,
A2) le mélange d'au moins un échantillon de microbiote fécal d'un sujet donneur avec un tampon salin comprenant éventuellement de l'iodixanol ou du 5- (N-2, 3-dihydroxypropylacetamido)-2, 4, 6-tri-iodo-N, N'-bis (2, 3 dihydroxypropyl) isophthalamide, en anaérobiose,
A3) optionnellement, le dépôt du mélange obtenu en A2) sous le gradient obtenu en A1),
A4) la centrifugation séquentielle à faible accélération en anaérobiose, ou l'ultracentrifugation du mélange obtenu en A2) ou A3),
A5) la récupération de l'anneau bactérien ou du surnageant formé à l'issue de l'étape A4), en anaérobiose,
A6) le mélange de l'anneau bactérien ou du surnageant récupéré en A5) avec le diluant.

6. Procédé selon l'une des revendications 1 à 5, **caractérisé en ce qu'**il comprend, comme étape A6), les étapes suivantes en anaérobiose :
a) centrifugation de l'anneau bactérien remis en suspension dans un tampon salin ou du surnageant obtenu en A5), à une accélération comprise entre 3000 et 4000xg pendant un temps compris entre 5 et 15 minutes, à une température comprise entre 20 et 30°C,
b) récupération du culot obtenu à l'issue de l'étape a), et remise en suspension dans un tampon salin, puis centrifugation à une accélération comprise entre 200 et 500xg pendant un temps compris entre 5 et 15 minutes, à une température comprise entre 20 et 30°C,
c) récupération du surnageant obtenu à l'issue de l'étape b), et remise en suspension dans un tampon salin, puis centrifugation à une accélération comprise entre 3000 et 4000xg pendant un temps compris entre 5 et 15 minutes, à une température comprise entre 20 et 30°C,
d) récupération du culot obtenu à l'issue de l'étape c), et
e) le mélange du culot récupéré en d) avec le diluant.

7. Procédé selon l'une des revendications 1 à 6, **caractérisé en ce que** la lyophilisation de l'étape B) est réalisée en conditions suivantes :
B1) la congélation du mélange obtenu en A) à une température inférieure à -50°C, de préférence comprise entre -70°C et -100°C,
B2) le chargement du mélange congelé obtenu en B1) dans un lyophilisateur pré-refroidi à une température comprise entre -50°C et -30°C, à pression atmosphérique, puis
B3) au moins une étape de dessiccation primaire du mélange chargé en B2) comprenant l'abaissement de la pression jusqu'à une valeur comprise entre 80 et 200 µbar, puis l'augmentation de la température des étagères jusqu'à une valeur comprise entre -20°C et +25°C, en appliquant une vitesse de chauffage comprise entre 0,2 et 0,5°C/min, puis
B4) la dessiccation secondaire du mélange obtenu en B3) comprenant l'abaissement de la pression à une valeur inférieure ou égale à 80 µbar, et l'élévation de la température des étagères à une valeur comprise entre +25°C et +35°C, à une vitesse de chauffage comprise entre 0,1 et 0,3°C/min, et l'y maintenir entre 8 et 15 heures.

8. Procédé selon l'une des revendications 5 à 7, **caractérisé en ce que** le tampon salin est une solution aqueuse d'HEPES comprenant du chlorure de sodium, de préférence à une concentration comprise entre 7 et 15g/l.

9. Procédé selon l'une des revendications 1 à 8, **caractérisé en ce qu'**il comprend les étapes suivantes:
A1) la préparation d'un gradient continu d'iodixanol ou de 5- (N-2, 3-dihydroxypropylacetamido)-2, 4, 6-tri-iodo-N, N'-bis (2, 3 dihydroxypropyl) isophthalamide formé par congélation-décongélation,
A2) le mélange d'au moins un échantillon de microbiote fécal d'un sujet donneur avec un tampon salin additionné d'une solution aqueuse comprenant de l'iodixanol ou du 5- (N-2, 3-dihydroxypropylacetamido)-2, 4, 6-tri-iodo-N, N'-bis (2, 3 dihydroxypropyl) isophthalamide, ledit tampon salin étant de préférence une solution aqueuse d'HEPES comprenant du chlorure de sodium, en anaérobiose,
A3) le dépôt du mélange obtenu en A2) sous le gradient obtenu en A1),
A4) l'ultracentrifugation du mélange obtenu en A3), pendant une durée comprise entre 40 et 50 minutes, à une température comprise entre 2°C et 6°C, à une vitesse comprise entre 13000 et 16000 x *g,*
A5) la récupération de l'anneau bactérien formé à l'issue de l'étape A4), en anaérobiose,
A6) le mélange de l'anneau bactérien récupéré en A5) avec le diluant, et
B) la congélation du mélange obtenu en A6) à une température inférieure à -50°C, de préférence comprise entre -70°C et -100°C, puis sa lyophilisation.

10. Procédé selon l'une des revendications 1 à 9, **caractérisé en ce que** la préparation d'un gradient continu d'iodixanol formé par congélation-décongélation de l'étape A1) est réalisée selon les étapes suivantes :
A1.a) congélation d'une solution d'iodixanol ou de 5- (N-2, 3-dihydroxypropylacetamido)-2, 4, 6-tri-iodo-N, N'-bis (2, 3 dihydroxypropyl) isophthalamide à une température comprise entre -70°C et -100°C pendant au moins 12 heures ; puis
A1.b) décongélation de la solution obtenue en A1.a) à température ambiante pendant 2 à 4 heures, afin d'obtenir un gradient continu d'iodixanol ou de 5- (N-2, 3-dihydroxypropylacetamido)-2, 4, 6-tri-iodo-N, N'-bis (2, 3 dihydroxypropyl) isophthalamide.

11. Procédé selon l'une des revendications 1 à 8 ou 10, **caractérisé en ce qu'**il comprend les étapes suivantes en anaérobiose:
A2) le mélange d'au moins un échantillon de microbiote fécal d'un sujet donneur avec un tampon salin,
A4) la centrifugation séquentielle à faible accélération du mélange obtenu en A2),
A5) la récupération du surnageant formé à l'issue de l'étape A4),
A6) le mélange du surnageant récupéré en A5) avec le diluant, et
B) la congélation du mélange obtenu en A6) à une température inférieure à -50°C, de préférence comprise entre -70°C et -100°C, puis sa lyophilisation.

12. Procédé selon la revendication 11, **caractérisé en ce que** la centrifugation séquentielle à faible accélération de l'étape A4) est réalisée à une accélération comprise entre 200 et 500xg pendant un temps compris entre 5 et 15 minutes, à une température comprise entre 20 et 30°C.

13. Lyophilisat de microbiote fécal d'un sujet donneur susceptible d'être obtenu par le procédé selon l'une des revendications 1 à 12, pour son utilisation dans la transplantation de microbiote fécal autologue ou allogénique, ou pour traiter les dysbioses intestinales.

14. Utilisation in vitro d'un lyophilisat susceptible d'être obtenu par le procédé selon l'une des revendications 1 à 12, comme outil de recherche, de préférence en génomique fonctionnelle, en métaprotéomique ou en immunologie.

## Patentansprüche

1. Verfahren zur Herstellung eines Lyophilisats der fäkalen Mikrobiota eines Spenders, welches die folgenden Schritte umfasst:
A) Mischen einer Probe der fäkalen Mikrobiota eines Spenders mit einem Verdünner, der eine wässrige Salzlösung ist, welche (i) mindestens ein aus Polyolen, Di- bis Pentasacchariden oder deren Mischungen ausgewähltes Gefrierschutzmittel und (ii) Maltodextrine umfasst, und
B) Einfrieren der in A) erhaltenen Mischung bei einer Temperatur unter -50 °C, die vorzugsweise zwischen -70 °C und -100 °C liegt, und ihre anschließende Gefriertrocknung.

2. Verfahren zur Herstellung eines Lyophilisats nach Anspruch 1, **dadurch gekennzeichnet, dass** der Verdünner eine wässrige Salzlösung ist, die als Gefrierschutzmittel mindestens Galactose-Lactose oder Trehalose umfasst.

3. Verfahren zur Herstellung eines Lyophilisats nach einem der Ansprüche 1 oder 2, **dadurch gekennzeichnet, dass** der Verdünner eine wässrige Salzlösung ist, die mindestens ein Gemisch von Maltodextrinen umfasst, vorzugsweise in einer zwischen 4 und 20% liegenden Menge, bezogen auf das Gesamtvolumen der Lösung.

4. Verfahren zur Herstellung eines Lyophilisats nach einem der Ansprüche 1 bis 3, **dadurch gekennzeichnet, dass** die Gesamtmenge an Gefrierschutzmittel in der wässrigen Salzlösung zwischen 3 und 30 Gewichts-%, bezogen auf das Gesamtvolumen der Lösung, liegt, vorzugsweise zwischen 4 und 20 Gewichts-%, bezogen auf das Gesamtvolumen der Lösung.

5. Verfahren nach einem der Ansprüche 1 bis 4, **dadurch gekennzeichnet, dass** der Schritt A) die folgenden Schritte umfasst:
A1) optional Herstellen eines kontinuierlichen, durch Einfrieren und Auftauen gebildeten Gradienten von lodixanol oder von 5-(N-2,3-dihydroxypropylacetamido)-2,4,6-tri-iodo-N, N'-bis(2,3-dihydroxypropyl)-isophthalamid,
A2) Mischen mindestens einer Probe der fäkalen Mikrobiota eines Spenders mit einem Salz-Puffer, der gegebenenfalls lodixanol oder 5-(N-2,3-dihydroxypropylacetamido)-2,4,6-tri-iodo-N,N'-bis(2,3-dihydroxypropyl)-isophthalamid umfasst, unter anaeroben Bedingungen,
A3) optional Unterschichten des in A1) erhaltenen Gradienten mit der in A2) erhaltenen Mischung,
A4) sequentielles Zentrifugieren bei geringer Beschleunigung unter anaeroben Bedingungen oder Ultrazentrifugieren der in A2) oder A3) erhaltenen Mischung,
A5) Gewinnen des am Ende des Schrittes A4) gebildeten Bakterienrings oder Überstands unter anaeroben Bedingungen,
A6) Mischen des in A5) gewonnenen Bakterienrings oder Überstands mit dem Verdünner.

6. Verfahren nach einem der Ansprüche 1 bis 5, **dadurch gekennzeichnet, dass** es als Schritt A6 die folgenden Schritte unter anaeroben Bedingungen umfasst:
a) Zentrifugieren des in einem Salz-Puffer resuspendierten Bakterienrings oder des in A5) erhaltenen Überstands bei einer zwischen 3000 und 4000 x g liegenden Beschleunigung während eines zwischen 5 und 15 Minuten liegenden Zeitraums bei einer zwischen 20 und 30 °C liegenden Temperatur,
b) Gewinnen des am Ende des Schrittes a) erhaltenen Bodensatzes und Resuspendieren in einem Salz-Puffer, anschließend Zentrifugieren bei einer zwischen 200 und 500 × g liegenden Beschleunigung während eines zwischen 5 und 15 Minuten liegenden Zeitraums bei einer zwischen 20 und 30 °C liegenden Temperatur,
c) Gewinnen des am Ende des Schrittes b) erhaltenen Überstands und Resuspendieren in einem Salz-Puffer, anschließend Zentrifugieren bei einer zwischen 3000 und 4000 × g liegenden Beschleunigung während eines zwischen 5 und 15 Minuten liegenden Zeitraums bei einer zwischen 20 und 30 °C liegenden Temperatur,
d) Gewinnen des am Ende des Schrittes c) erhaltenen Bodensatzes, und
e) Mischen des in d) gewonnenen Bodensatzes mit dem Verdünner.

7. Verfahren nach einem der Ansprüche 1 bis 6, **dadurch gekennzeichnet, dass** die Gefriertrocknung von Schritt B) unter den folgenden Bedingungen durchgeführt wird:
B1) Einfrieren der in A) erhaltenen Mischung bei einer Temperatur unter -50 °C, die vorzugsweise zwischen -70 °C und -100 °C liegt,
B2) Einfüllen der in B1) erhaltenen gefrorenen Mischung in einen auf eine zwischen -50 °C und -30 °C liegende Temperatur vorgekühlten Gefriertrockner bei Atmosphärendruck, anschließend
B3) mindestens ein primärer Trocknungsschritt der in B2) eingefüllten Mischung, welcher umfasst: Absenken des Drucks bis auf einen zwischen 80 und 200 µbar liegenden Wert, anschließend Erhöhen der Temperatur der Stellflächen bis auf einen zwischen -20 °C und +25 °C liegenden Wert unter Anwendung einer zwischen 0,2 und 0,5 °C/min liegenden Heizgeschwindigkeit, anschließend
B4) sekundäres Trocknen der in B3) erhaltenen Mischung, welches umfasst: Absenken des Drucks auf einen Wert, der kleiner als oder gleich 80 µbar ist, und Erhöhen der Temperatur der Stellflächen auf einen zwischen +25 °C und +35 °C liegenden Wert mit einer zwischen 0,1 und 0,3 °C/min liegenden Heizgeschwindigkeit, und sie zwischen 8 und 15 Stunden dort zu halten.

8. Verfahren nach einem der Ansprüche 5 bis 7, **dadurch gekennzeichnet, dass** der Salz-Puffer eine wässrige HEPES-Lösung ist, die Natriumchlorid umfasst, vorzugsweise in einer zwischen 7 und 15 g/l liegenden Konzentration.

9. Verfahren nach einem der Ansprüche 1 bis 8, **dadurch gekennzeichnet, dass** es die folgenden Schritte umfasst:
A1) Herstellen eines kontinuierlichen, durch Einfrieren und Auftauen gebildeten Gradienten von lodixanol oder von 5-(N-2,3-dihydroxypropylacetamido)-2,4,6-tri-iodo-N, N'-bis(2,3-dihydroxypropyl)-isophthalamid,
A2) Mischen mindestens einer Probe der fäkalen Mikrobiota eines Spenders mit einem Salz-Puffer, welcher mit einer wässrigen Lösung versetzt ist, die lodixanol oder 5-(N-2,3-dihydroxypropylacetamido)-2,4,6-tri-iodo-N,N'-bis(2,3-dihydroxypropyl)-isophthalamid umfasst, wobei der Salz-Puffer vorzugsweise eine wässrige HEPES-Lösung ist, die Natriumchlorid umfasst, unter anaeroben Bedingungen,
A3) Unterschichten des in A1) erhaltenen Gradienten mit der in A2) erhaltenen Mischung,
A4) Ultrazentrifugieren der in A3) erhaltenen Mischung während einer zwischen 40 und 50 Minuten liegenden Dauer bei einer zwischen 2 °C und 6 °C liegenden Temperatur, bei einer zwischen 13000 und 16000 × g liegenden Geschwindigkeit,
A5) Gewinnen des am Ende des Schrittes A4) gebildeten Bakterienrings unter anaeroben Bedingungen,
A6) Mischen des in A5) gewonnenen Bakterienrings mit dem Verdünner, und
B) Einfrieren der in A6) erhaltenen Mischung bei einer Temperatur unter -50 °C, die vorzugsweise zwischen -70 °C und -100 °C liegt, und ihre anschließende Gefriertrocknung.

10. Verfahren nach einem der Ansprüche 1 bis 9, **dadurch gekennzeichnet, dass** das Herstellen eines kontinuierlichen, durch Einfrieren und Auftauen gebildeten lodixanol-Gradienten von Schritt A1) gemäß den folgenden Schritten durchgeführt wird:
A1.a) Einfrieren einer Lösung von lodixanol oder von 5-(N-2,3-dihydroxypropylacetamido)-2,4,6-tri-iodo-N,N'-bis(2,3-dihydroxypropyl)-isophthalamid bei einer zwischen -70 °C und -100 °C liegenden Temperatur während mindestens 12 Stunden; anschließend
A1.b) Auftauen der in A1.a) erhaltenen Lösung auf Raumtemperatur während 2 bis 4 Stunden, um einen kontinuierlichen Gradienten von lodixanol oder von 5-(N-2,3-dihydroxypropylacetamido)-2,4,6-tri-iodo-N,N'-bis(2,3-dihydroxypropyl)-isophthalamid zu erhalten.

11. Verfahren nach einem der Ansprüche 1 bis 8 oder 10, **dadurch gekennzeichnet, dass** es die folgenden Schritte unter anaeroben Bedingungen umfasst:
A2) Mischen mindestens einer Probe der fäkalen Mikrobiota eines Spenders mit einem Salz-Puffer,
A4) sequentielles Zentrifugieren der in A2) erhaltenen Mischung bei geringer Beschleunigung,
A5) Gewinnen des am Ende des Schrittes A4) gebildeten Überstands,
A6) Mischen des in A5) gewonnenen Überstands mit dem Verdünner, und
B) Einfrieren der in A6) erhaltenen Mischung bei einer Temperatur unter -50 °C, die vorzugsweise zwischen -70 °C und -100 °C liegt, und ihre anschließende Gefriertrocknung.

12. Verfahren nach Anspruch 11, **dadurch gekennzeichnet, dass** das sequentielle Zentrifugieren bei geringer Beschleunigung von Schritt A4) bei einer zwischen 200 und 500 × g liegenden Beschleunigung, während eines zwischen 5 und 15 Minuten liegenden Zeitraums, bei einer zwischen 20 und 30 °C liegenden Temperatur durchgeführt wird.

13. Lyophilisat der fäkalen Mikrobiota eines Spenders, erhältlich durch das Verfahren nach einem der Ansprüche 1 bis 12, zur Verwendung in der autologen oder allogenen fäkalen Mikrobiota-Transplantation oder zur Behandlung von Darmdysbiosen.

14. Verwendung eines Lyophilisats, welches durch das Verfahren nach einem der Ansprüche 1 bis 12 erhältlich ist, als Forschungswerkzeug in vitro, vorzugsweise in der funktionellen Genomik, in der Metaproteomik oder in der Immunologie.

## Claims

1. A method of preparing a lyophilizate of faecal microbiota from a donor subject, comprising the following steps:
A) mixing a sample of faecal microbiota from a donor subject with a diluent that is a saline aqueous solution comprising (i) at least one cryoprotectant chosen from polyols, di- to pentasaccharides or mixtures thereof, and (ii) maltodextrins, and
B) freezing the mixture obtained in A) at a temperature less than -50°C, preferably comprised between -70°C and -100°C, then lyophilizing it.

2. A method of preparing a lyophilizate according to claim 1 , **characterized in that** the diluent is a saline aqueous solution comprising at least galactose-lactose or trehalose as cryoprotectant.

3. A method of preparing a lyophilizate according to one of claims 1 or 2, **characterized in that** the diluent is a saline aqueous solution comprising at least a mixture of maltodextrins, preferably in an amount comprised between 4 and 20% relative to the total volume of the solution.

4. A method of preparing a lyophilizate according to one of claims 1 to 3, **characterized in that** the total amount of cryoprotectant in the saline aqueous solution is comprised between 3 and 30% by weight relative to the total volume of the solution, preferably between 4 and 20% by weight relative to the total volume of the solution.

5. A method according to any one of claims 1 to 4, **characterized in that** step A) comprises the following steps:
A1) optionally, preparing a continuous gradient of iodixanol or 5-(N-2, 3-dihydroxypropylacetamido)-2, 4, 6-tri-iodo-N, N'-bis (2, 3 dihydroxypropyl) isophthalamide formed by freeze-thaw,
A2) mixing at least one faecal microbiota sample from a donor subject with a saline buffer optionally comprising iodixanol or 5-(N-2, 3-dihydroxypropylacetamido)-2, 4, 6-tri-iodo-N, N'-bis (2, 3 dihydroxypropyl) isophthalamide, under anaerobiosis,
A3) optionally, forming a deposit of the mixture obtained in A2) under the gradient obtained in A1),
A4) sequentially centrifuging at low acceleration under anaerobiosis, or ultracentrifuging the mixture obtained in A2) or A3),
A5) recovering the bacterial ring or the supernatant formed on finishing step A4), under anaerobiosis,
A6) mixing the bacterial ring or the supernatant recovered in A5) with the diluent.

6. A method according to one of claims 1 to 5, **characterized in that** it comprises, as step A6), the following steps under anaerobiosis:
a) centrifuging the bacterial ring resuspended in a saline buffer or the supernatant obtained in A5), at an acceleration comprised between 3000 and 4000xg for a time comprised between 5 and 15 minutes, at a temperature comprised between 20 and 30°C,
b) recovering the pellet obtained on finishing step a), and resuspending in a saline buffer, then centrifuging at an acceleration comprised between 200 and 500xg for a time comprised between 5 and 15 minutes, at a temperature comprised between 20 and 30°C,
c) recovering the supernatant obtained on finishing step b), and resuspending in a saline buffer, then centrifuging at an acceleration comprised between 3000 and 4000xg for a time comprised between 5 and 15 minutes, at a temperature comprised between 20 and 30°C,
d) recovering the pellet obtained on finishing step c), and
e) mixing the pellet recovered in d) with the diluent.

7. A method according to one of claims 1 to 6, **characterized in that** the lyophilization of step B) is carried out in the following conditions:
B1) freezing the mixture obtained in A) at a temperature less than -50°C, preferably comprised between -70°C and -100°C,
B2) loading the frozen mixture obtained in B1) into a precooled lyophilizer to a temperature comprised between -50°C and -30°C, at atmospheric pressure, then
B3) at least one step of primary drying of the mixture loaded at B2) comprising lowering the pressure to a value comprised between 80 and 200 µbar, then increasing the temperature of the shelves to a value comprised between -20°C and +25°C, while applying a heating rate comprised between 0.2 and 0.5 °C/min, then
B4) secondary drying of the mixture obtained at B3) comprising lowering the pressure to a value less than or equal to 80 µbar, and raising the temperature of the shelves to a value comprised between +25°C and +35°C, at a heating rate comprised between 0.1 and 0.3 °C/min, and maintaining it there for between 8 and 15 hours.

8. A method according to one of claims 5 to 7, **characterized in that** the saline buffer is an aqueous solution of HEPES comprising sodium chloride, preferably at a concentration comprised between 7 and 15g/l.

9. A method according to one of claims 1 to 8, **characterized in that** it comprises the following steps:
A1) preparing a continuous gradient of iodixanol or 5-(N-2, 3-dihydroxypropylacetamido)-2, 4, 6-tri-iodo-N, N'-bis (2, 3 dihydroxypropyl) isophthalamide formed by freeze-thaw,
A2) mixing at least one faecal microbiota sample from a donor subject with a saline buffer added to an aqueous solution comprising iodixanol or 5-(N-2, 3-dihydroxypropylacetamido)-2, 4, 6-tri-iodo-N, N'-bis (2, 3 dihydroxypropyl) isophthalamide, said saline buffer preferably being an aqueous solution of HEPES comprising sodium chloride, under anaerobiosis,
A3) forming a deposit of the mixture obtained in A2) under the gradient obtained in A1),
A4) ultracentrifuging the mixture obtained in A3), for a time comprised between 40 and 50 minutes, at a temperature comprised between 2°C and 6°C, at a speed comprised between 13000 and 16000 x *g,*
A5) recovering the bacterial ring formed on finishing step A4), under anaerobiosis,
A6) mixing the bacterial ring recovered in A5) with the diluent, and
B) freezing the mixture obtained in A6) at a temperature less than -50°C, preferably comprised between -70°C and -100°C, then lyophilizing it.

10. A method according to one of claims 1 to 9, **characterized in that** the preparing of a continuous gradient of iodixanol formed by freeze-thaw in step A1) is carried out according to the following steps:
A1.a) freezing a solution of iodixanol or 5-(N-2, 3-dihydroxypropylacetamido)-2, 4, 6-tri-iodo-N, N'-bis (2, 3 dihydroxypropyl) isophthalamide at a temperature comprised between -70°C and -100°C for at least 12 hours; then
A1.b) thawing the solution obtained in A1.a) at an ambient temperature for 2 to 4 hours, in order to obtain a continuous gradient of iodixanol or of 5-(N-2, 3-dihydroxypropylacetamido)-2, 4, 6-tri-iodo-N, N'-bis (2, 3 dihydroxypropyl) isophthalamide.

11. A method according to one of claims 1 to 8 or 10, **characterized in that** it comprises the following steps under anaerobiosis:
A2) mixing at least one sample of faecal microbiota from a donor subject with saline buffer,
A4) sequentially centrifuging at low acceleration the mixture obtained in A2),
A5) recovering the supernatant formed on finishing step A4),
A6) mixing the supernatant recovered in A5) with the diluent, and
B) freezing the mixture obtained in A6) at a temperature less than -50°C, preferably comprised between -70°C and -100°C, then lyophilizing it.

12. A method according to claim 11, **characterized in that** the sequential centrifugation at low acceleration of step A4) is carried out at an acceleration comprised between 200 and 500xg for a time comprised between 5 and 15 minutes, at a temperature comprised between 20 and 30°C,

13. A lyophilizate of fecal microbiota from a donor subject obtainable by the method according to one of claim 1 to 12, for use in the transplantation of autologous or allogenic faecal microbiota, or for treating intestinal dysbioses.

14. The use in vitro of a lyophilizate obtainable by the method according to one of claim 1 to 12, as a research tool, preferably in functional genomics, metaproteomics or immunology.
